# EUROPÄISCHE PATENTSCHRIFT

(11) **EP 3 865 163 B1**
(45) Veröffentlichungstag und Bekanntmachung des Hinweises auf die Patenterteilung: **01.04.2026**
(21) Anmeldenummer: 21020043.2
(22) Anmeldetag: 02.02.2021
(51) Int. Cl.: A61M 16/00, A61B 5/087, A61B 5/091, A61B 5/097, A61B 5/00

(54) **AUSWERTESYSTEM FÜR EIN BEATMUNGSGERÄT UND VERFAHREN**
EVALUATION SYSTEM FOR A VENTILATOR AND METHOD
SYSTÈME D'ÉVALUATION DOTÉ D'UN APPAREIL RESPIRATOIRE ET PROCÉDÉ

(30) Priorität: 13.02.2020 DE 102020103837
(43) Veröffentlichungstag der Anmeldung: 18.08.2021
(73) Patentinhaber: Löwenstein Medical Technology S.A., 2557 Luxembourg (LU)
(72) Erfinder: Verhoeven, Jan, 76275 Ettlingen (DE); Dömer, Benno, 76275 Ettlingen (DE); Alshut, Rüdiger, 76131 Karlsruhe (DE); Schwaibold, Matthias, Karlsruhe (DE)
(74) Vertreter: Löwenstein Medical IP

(56) Entgegenhaltungen:
- EP-A2- 2 216 063
- JP-A- 2012 083 886
- US-A1- 2011 029 248
- US-A1- 2014 330 155

## Beschreibung

Die vorliegende Erfindung betrifft ein Auswertesystem für wenigstens ein zum maschinellen Beatmen eines Patienten vorgesehenes Beatmungsgerät sowie ein Verfahren zum Betreiben eines solchen Auswertesystems. Das Auswertesystem umfasst wenigstens eine Auswerteeinrichtung, welche mittels wenigstens eines Sensormittels wenigstens einen Beatmungsparameter über die Zeit registriert.

Um den Erfolg bzw. die Qualität einer maschinellen Beatmung kontrollieren zu können, wird in der Regel eine Auswertung von Daten vorgenommen, welche erhöhte Fachkenntnisse des Personals erfordert. Meist ist für eine solche Bewertung eine zeitaufwendige Auswertung von zahlreichen Messsignalen bzw. Beatmungsparametern und deren Verläufen erforderlich. EP 2216063 A2 offenbart eine Beatmungsvorrichtung, die während der Beatmung Parameter überwacht und bewertet und eine Komplikationskontrolle beinhaltet. US 2011/0029248 A1 offenbart eine Vorrichtung zur Vorhersage der Atemstabilität von Patienten, die auf unterschiedlichen Atmungs- und Herz-Parametern basiert. US 2014/330155 A1 offenbart ein System zur Überwachung der Atmungsstabilität und -wirksamkeit.

Besonders problematisch ist die Kontrolle der Beatmungsqualität dann, wenn der Patient zu Hause beatmet wird. Denn im Gegensatz zu einer Klinik ist in der häuslichen Pflege eine Kontrolle durch häufigeres Besuchen des Patienten nur schwer umsetzbar.

Demgegenüber ist es die Aufgabe der vorliegenden Erfindung, die Überwachung und Bewertung der Beatmung zu verbessern.

Insbesondere soll auch unerfahrenen Benutzern bzw. Patienten eine zuverlässige Einschätzung der Beatmungsqualität ermöglicht werden. Vorzugsweise soll dabei die Überwachung auch für das Fachpersonal komfortabler und zugleich zuverlässiger erfolgen können.

Diese Aufgabe wird gelöst mit einem Auswertesystem des Anspruchs 1 sowie mit einem Verfahren gemäß Anspruch 18.

Weiterbildungen und vorteilhafte Ausgestaltungen sind Gegenstand der Unteransprüche. Weitere Vorteile und Merkmale ergeben sich aus der allgemeinen Beschreibung und der Beschreibung der Ausführungsbeispiele.

Das erfindungsgemäße Auswertesystem dient zur Auswertung wenigstens eines während einer Beatmung sensorisch erfassten Beatmungsparameters und/oder anderer Behandlungsdaten. Das Auswertesystem ist zur Verwendung mit wenigstens einem Beatmungsgerät vorgesehen oder umfasst wenigstens ein solches. Das Beatmungsgerät dient insbesondere zur maschinellen Beatmung eines Patienten. Erfindungsgemäß kann darunter eine kontrollierte Beatmung und/oder eine assistierte Beatmung verstanden werden. Das Auswertesystem umfasst wenigstens eine Auswerteeinrichtung, welche dazu geeignet und ausgebildet ist, wenigstens zwei unterschiedliche mittels wenigstens eines Sensormittels erfassten Beatmungsparameter über die Zeit zu registrieren, wobei die zwei unterschiedlichen Beatmungsparameter ausgewählt sind aus der Gruppe Atemfrequenz, Atemvolumen, Atemgasfluss, Atemgasdruck, dadurch gekennzeichnet, dass die Auswerteeinrichtung dazu geeignet und ausgebildet ist, aus dem zeitlichen Verlauf der wenigstens zwei unterschiedlichen Beatmungsparameter wenigstens eine Stabilitätsrate zu berechnen, und aus der Stabilitätsrate wenigstens eine Kennzahl für eine Atemstabilität des Patienten zu ermitteln, sodass die Kennzahl eine Bewertung der Qualität der Beatmung während eines Zeitraums bereitstellt. Dadurch stellt die Kennzahl vorzugsweise eine Bewertung der Qualität der Beatmung während eines Zeitraums, insbesondere eines definierten Zeitraums und beispielsweise eines Zeitraums von wenigstens 2 Stunden oder mehr, bereit. Erfindungsgemäß ist die Auswerteeinrichtung dazu geeignet und ausgebildet, die Kennzahl während der Beatmung zu ermitteln und in bestimmten Zeitabständen und fortlaufend zu aktualisieren, sodass die Kennzahl zurückliegende Zeiträume und die diesen Zeiträumen zugeordneten Werte für die Kennzahl wenigstens teilweise berücksichtigt.

Die Kennzahl wird insbesondere durch eine statistische Auswertung eines über die Zeit registrierten Atemmusters ermittelt. Das Atemmuster wird dabei durch den zeitlichen Verlauf des wenigstens einen Beatmungsparameters bereitgestellt. Die Kennzahl kann auch als Indikator bzw. Index bezeichnet werden. Unter einer Kennzahl wird erfindungsgemäß nicht nur eine Zahl, sondern insbesondere auch ein Symbol oder dergleichen verstanden.

Das erfindungsgemäße Auswertesystem bietet viele Vorteile. Einen erheblichen Vorteil bietet die Bereitstellung der Kennzahl durch das Auswertesystem. Dadurch erhält ein Benutzer durch Betrachtung der Kennzahl und insbesondere von lediglich einer einzigen Kennzahl eine besonders aussagekräftige und beispielsweise eine statistische Bewertung der Qualität der durchgeführten Beatmung. So kann eine besonders zuverlässige Bewertung der Beatmungsqualität sozusagen auf einen Blick erfolgen. Zudem ermöglicht die Erfindung auch unerfahrenen Benutzern bzw. Patienten eine besonders hilfreiche Einschätzung der Beatmung. Des Weiteren kann bei der Erfindung auch in der häuslichen Pflege bzw. der Heimbeatmung eine sichere und komfortable Überwachung und Bewertung der Beatmung erfolgen, auch wenn das Fachpersonal nicht oder nur selten vor Ort ist. Besonders vorteilhaft kann die Erfindung zum Beispiel in der Telemedizin eingesetzt werden. Zudem bietet die Erfindung eine erhebliche Zeitersparnis, da eine oft mühselige und zeitintensive Auswertung von Beatmungsparametern über längere Zeiträume nicht mehr oder nur noch dann notwendig ist, wenn die Kennzahl dazu veranlasst.

Einen erheblichen Vorteil bietet die Bereitstellung einer Kennzahl für die Qualität der Beatmung oder vorzugsweise eine Bewertung der Qualität der Beatmung während eines Zeitraums, da eine optimale Atemstabilität ohne Asynchronien oder andere Störungen für den Patienten medizinisch vorteilhaft ist und/oder auch der Energieverbrauch oder der Regelungsaufwand des Beatmungsgerätes minimiert und damit die Akkulaufzeit des Beatmungsgerätes verlängert wird oder bedingt durch den geringeren Regelungsaufwand der Verschleiß beispielsweise an dem Elektrogebläse minimiert ist.

Vorzugsweise ist die Auswerteeinrichtung dazu geeignet und ausgebildet, die Kennzahl als wenigstens eine Zahl und/oder wenigstens eine Grafik und/oder wenigstens ein Symbol und/oder wenigstens ein Wort auszugeben und insbesondere mit wenigstens einer Anzeigeeinrichtung anzuzeigen. Die Zahl kann mit oder ohne Einheiten ausgegeben werden. In allen Ausgestaltungen ist es möglich, dass die Anzeigeeinrichtung vom Auswertesystem und/oder vom Beatmungsgerät und/oder von anderen Geräten und beispielsweise einem PC bzw. Rechner oder einem mobilen Endgerät oder dergleichen bereitgestellt wird. Es ist möglich vorteilhaft, dass die Kennzahl nur durch eine einzige Zahl und/oder Grafik und/oder nur durch ein einziges Symbol und/oder Wort ausgegeben wird.

In einer vorteilhaften Ausgestaltung entspricht die ermittelte Kennzahl wenigstens einem Wert auf einer Skala oder umfasst einen solchen. Insbesondere ist einem Ende der Skala eine höhere bzw. hohe und insbesondere maximale Atemstabilität und einem anderen Ende der Skala eine geringere bzw. geringe und insbesondere minimale Atemstabilität zugeordnet. Dabei ist die Auswerteeinrichtung bevorzugt dazu geeignet und ausgebildet, den Wert und/oder die Skala mit dem darauf positionierten Wert auszugeben und vorzugsweise auf der Anzeigeeinrichtung anzuzeigen. Besonders bevorzugt ist eine Skala zwischen null und 100 oder eins und 100 vorgesehen. Möglich ist auch, dass eine Skala zwischen null und zehn oder eins und zehn vorgesehen ist. Solche Zahlenbereiche ermöglichen eine besonders anschauliche Skala. Möglich sind auch Skalen mit anderen Zahlenbereichen.

Insbesondere ist eine dimensionslose Skala vorgesehen. Möglich ist auch eine Skala mit Einheiten. Die ermittelte Kennzahl kann auch wenigstens einem Wertebereich auf der Skala entsprechen. Möglich ist auch, dass die Skala und/oder der Wert durch wenigstens ein Wort und/oder wenigstens ein Symbol und/oder wenigstens eine Grafik oder dergleichen definiert sind. Beispielsweise kann eine Skala von sehr gut über gut und mittel bis schlecht oder dergleichen vorgesehen sein. Als Symbole können beispielsweise Pfeile vorgesehen sein, welche nach unten und nach oben und horizontal ausgerichtet sind. Möglich sind auch andere Symbole. Es ist möglich und vorteilhaft, dass die Skala grafisch aufbereitet ist.

In allen Ausgestaltungen ist es besonders vorteilhaft und bevorzugt, dass die Auswerteeinrichtung dazu geeignet und ausgebildet ist, aus dem zeitlichen Verlauf des Beatmungsparameters wenigstens eine Stabilitätsrate zu berechnen. Vorzugsweise kann die Auswerteeinrichtung die Kennzahl wenigstens aus der Stabilitätsrate ermitteln. Insbesondere wird die Kennzahl mittels wenigstens einer Zuordnungsfunktion aus der Stabilitätsrate berechnet. Die Stabilitätsrate entspricht insbesondere einem zeitlichen Verlauf der Stabilität und/oder der Variabilität der Atmung. Beispielsweise ist die Stabilitätsrate hoch, wenn der wenigstens eine Beatmungsparameter über die Zeit stabil ist bzw. wenig schwankt. Beispielsweise ist die Stabilitätsrate niedrig, wenn der wenigstens eine Beatmungsparameter instabil ist bzw. über die Zeit stärker schwankt.

Die Stabilitätsrate kann auch als eine Variabilitätsrate ausgebildet sein. Es ist möglich, dass die Stabilitätsrate dann eine Variabilität der Beatmung bzw. Atmung beschreibt und insbesondere dem Kehrwert der Kennzahl entspricht. Dann sind die zuvor genannten Beispiele analog umgekehrt (ein hoher Wert entspricht einer hohen Variabilität bzw. einer geringen Stabilität). Es kann auch eine andere geeignete Zuordnungsfunktion zwischen Stabilitätsrate und Kennzahl vorgesehen sein.

In einer besonders vorteilhaften Ausgestaltung ist die Auswerteeinrichtung dazu geeignet und ausgebildet, wenigstens einem Zeitraum, in welchem die Stabilitätsrate einen Schwellenwert für die Stabilität überschreitet, eine Kennzahl mit einem für eine maximale Atemstabilität vorgesehenen Wert zuzuordnen. Möglich und vorteilhaft ist auch, dass die Auswerteeinrichtung dazu geeignet und ausgebildet ist, wenigstens einem Zeitraum, in welchem die Stabilitätsrate einen Schwellenwert für die Instabilität überschreitet, eine Kennzahl mit einem für eine minimale Atemstabilität vorgesehenen Wert zuzuordnen. Der für eine maximale Atemstabilität vorgesehene Wert ist insbesondere der höchste Wert der für die Kennzahl vorgesehenen Skala. Der für eine minimale Atemstabilität vorgesehene Wert ist insbesondere der niedrigste Wert der für die Kennzahl vorgesehenen Skala.

Es ist ebenfalls vorteilhaft und bevorzugt, dass die Auswerteeinrichtung dazu geeignet und ausgebildet ist, wenigstens einem Zeitraum, in welchem die Stabilitätsrate zwischen dem Schwellenwert für die Stabilität und dem Schwellenwert für die Instabilität liegt, eine Kennzahl mit wenigstens einem Wert zuzuordnen. Dabei ist die Auswerteinrichtung insbesondere dazu geeignet und ausgebildet, den Wert mittels wenigstens einer Zuordnungsfunktion und vorzugsweise mittels einer linearen Interpolation aus der Stabilitätsrate zu berechnen. Möglich sind auch andere Arten von Zuordnungsfunktionen.

Die Auswerteeinrichtung ist vorzugsweise dazu geeignet und ausgebildet, die Kennzahl während der Beatmung zu ermitteln und insbesondere in bestimmten Zeitabständen und/oder fortlaufend zu aktualisieren. So kann beispielsweise eine Kennzahl, welche eine geringe Stabilität anzeigt, nach einer längeren Phase mit einer guten Atemstabilität einen Wert annehmen, welcher die verbesserte Atemstabilität berücksichtigt. Insbesondere wird die aktualisierte Kennzahl aus vorangegangenen Kennzahlen gemittelt und/oder in einer anderen Weise statistisch abgeleitet.

Es ist möglich und vorteilhaft, dass für wenigstens zwei oder mehr unterschiedliche Beatmungsparameter unterschiedliche Stabilitätsraten und/oder Kennzahlen berechnet werden. Vorzugsweise werden die jeweiligen Stabilitätsraten und/oder die jeweiligen Kennzahlen dann miteinander verglichen. Insbesondere kann die Auswerteinrichtung über den Vergleich wenigstens eine Atemstörung und/oder wenigstens ein Atemereignis im Atemmuster erkennen. Beispielsweise können so Asynchronien, Apnoen und/oder Flusslimitationen erkannt werden.

Die Auswerteeinrichtung kann dazu geeignet und ausgebildet sein, zur Ermittlung der Kennzahl wenigstens einen statistischen Kennwert für eine Tendenz des zeitlichen Verlaufs des Beatmungsparameters heranzuziehen. Vorzugsweise ist der statistische Kennwert ein Mittelwert und besonders bevorzugt ein gleitender Mittelwert oder umfasst wenigstens einen solchen. Möglich sind auch andere statistische Kennwerte für die Tendenz.

In einer vorteilhaften Ausgestaltung ist die Auswerteeinrichtung dazu geeignet und ausgebildet, die Kennzahl wenigstens aus wenigstens einem statistischen Streuungsmaß für eine Verteilung von Werten des Beatmungsparameters im zeitlichen Verlauf des Beatmungsparameters zu ermitteln. Dabei ist das statistische Streuungsmaß vorzugsweise eine Varianz oder umfasst wenigstens eine solche. Möglich sind auch andere geeignete statistische Streuungsmaße.

Es ist möglich, dass die Auswerteeinrichtung dazu geeignet und ausgebildet ist, wenigstens eine Tendenz für eine erfolgte und/oder zukünftige Entwicklung der Kennzahl zu berechnen und insbesondere auszugeben. Beispielsweise kann angezeigt werden, ob sich die Kennzahl im Verlauf eines zurückliegenden Zeitraums in Richtung höherer Atemstabilität oder geringerer Atemstabilität entwickelt hat. Eine solche Tendenz kann beispielsweise durch ein Symbol und/oder eine Grafik und zum Beispiel einen Pfeil dargestellt werden.

In allen Ausgestaltungen ist es bevorzugt, dass der wenigstens eine Beatmungsparameter, welcher zur Ermittlung der Kennzahl herangezogen wird, wenigstens eine Atemfrequenz und/oder ein Atemvolumen bzw. Atemzugvolumen und/oder einen Atemgasfluss und/oder einen Atemgasdruck oder eine Kombination solcher Parameter betrifft. Insbesondere ist die Auswerteeinrichtung dazu geeignet und ausgebildet, wenigstens einen und vorzugsweise wenigstens zwei oder mehr oder alle der vorgenannten Beatmungsparameter zur Berechnung der Kennzahl und/oder der Stabilitätsrate heranzuziehen. Die Auswerteeinrichtung kann auch gezielt wenigstens einen oder mehrere Beatmungsparameter unberücksichtigt lassen. Die Auswerteeinrichtung kann auch wenigstens eine unterschiedliche Gewichtung bestimmter Beatmungsparameter vornehmen. Die Auswahl bzw. Gewichtung der Beatmungsparameter kann beispielsweise von einer am Beatmungsgerät eingestellten Art der Beatmung durch die Auswerteeinrichtung vorgenommen werden.

In einer vorteilhaften Ausgestaltung ist die Auswerteeinrichtung dazu geeignet und ausgebildet, aufgrund wenigstens einer der folgenden Ursachen die Kennzahl in Richtung schlechter Atemstabilität zu verändern: Asynchronität zwischen Beatmungsgerät und Patient; Auftreten von partiellen oder vollständigen intermittierenden Atemwegsobstruktionen; Insomnie; Beschwerden durch Maske und/oder Therapiedruck; Atemnot; starke Sekretbildung; Hustenreiz; instabilen Atemantrieb; periodische Atmung; starke Leckagen.

In einer vorteilhaften Weiterbildung ist die Auswerteeinrichtung dazu geeignet und ausgebildet, wenigstens eine Aktion auszuführen, wenn die Kennzahl und/oder die Stabilitätsrate in wenigstens einem definierten kritischen Wertebereich für die Atemstabilität liegt und/oder eine Tendenz dahin aufweist. Es ist möglich, dass die Aktion ausgeführt wird, wenn die Kennzahl unterhalb oder oberhalb eines definierten Schwellenwerts liegt oder eine Tendenz dahin aufweist.

Vorzugsweise umfasst die Aktion wenigstens eine Ausgabe wenigstens eines Warnhinweises und/oder wenigstens eines Alarms und/oder wenigstens einer Handlungsbeschreibung. Die Aktion kann teilweise akustisch und/oder optisch und/oder haptisch erfolgen. Die Aktion kann wenigstens eine Ausgabe der Kennzahl in Kombination mit wenigstens einer akustischen und/oder optischen und/oder haptischen Hervorhebung umfassen. Möglich und bevorzugt ist, dass die Aktion auf wenigstens einem mit der Auswerteeinrichtung gekoppelten Gerät ausgeführt wird. Beispielsweise kann die Aktion auf einem mobilen Endgerät und/oder auf einem Rechner bzw. PC und/oder dem Beatmungsgerät und/oder einem anderen Gerät ausgeführt werden.

In einer vorteilhaften Ausgestaltung umfasst die Aktion wenigstens eine Ansteuerung des Beatmungsgerätes. Insbesondere betrifft die Ansteuerung wenigstens einen Beatmungsparameter. Insbesondere wird das Beatmungsgerät durch die Aktion so angesteuert, dass eine Veränderung der Kennzahl in Richtung einer höheren und gegebenenfalls auch geringeren Atemstabilität erreichbar ist. Insbesondere regelt das Beatmungsgerät dazu wenigstens einen Beatmungsparameter auf wenigstens einen in der Aktion hinterlegten Sollwert.

Es ist vorteilhaft und bevorzugt, dass sowohl wenigstens ein kritischer Wertebereich für eine zu hohe als auch wenigstens ein kritischer Wertebereich für eine zu niedrige Atemstabilität vorgesehen sind. Das hat den Vorteil, dass auch eine zu starre Atmung bzw. eine zu hohe Atemstabilität zu einer Aktion führen kann. So können auch Atemstörungen beispielsweise durch einen Alarm angezeigt werden, welche mit einer eher hohen Atemstabilität einhergehen.

Die Auswerteeinrichtung ist insbesondere dazu geeignet und ausgebildet, die Kennzahl in Kombination mit wenigstens einem Wert für wenigstens einen Beatmungsparameter auszugeben. Ein solcher Wert ist vorzugsweise statistisch über die Zeit aufbereitet. Beispielsweise kann ein Mittelwert des Beatmungsparameters in Kombination mit der Kennzahl ausgegeben werden. Beispielsweise wird die Kennzahl in Kombination mit der Atemfrequenz und/oder der Atemtiefe und/oder dem Atemgasdruck und/oder dem Atemgasfluss und/oder dem Atemzugvolumen ausgegeben. Die Atemtiefe entspricht insbesondere dem Tidalvolumen bzw. einem Quotienten aus dem Tidalvolumen und/oder einem Quotienten aus Frequenz und Tidalvolumen. Die Kennzahl kann auch in Kombination mit wenigstens einer anderen Kennzahl bzw. mit anderen Indizes kombiniert werden, z. B. um einen Interventionsbedarf zur Optimierung der Beatmung anzuzeigen oder wegen einer akuten Verschlechterung der Erkrankung.

Es ist besonders bevorzugt und vorteilhaft, dass die Auswerteeinrichtung dazu geeignet und ausgebildet ist, das Beatmungsgerät in Abhängigkeit der ermittelten Kennzahl anzusteuern und vorzugsweise wenigstens eine Steuergröße des Beatmungsgerätes anzupassen, um insbesondere eine Veränderung der Kennzahl in Richtung einer höheren Atemstabilität zu erreichen. Insbesondere stehen die Auswerteeinrichtung und das Beatmungsgerät in Wirkverbindung. Insbesondere kann die Auswerteeinrichtung das Beatmungsgerät unter Berücksichtigung der Kennzahl steuern bzw. regeln und beispielsweise auf einen Sollwert wenigstens eines Beatmungsparameters einstellen.

Insbesondere ist die Auswerteeinrichtung dazu geeignet und ausgebildet, zur Erzielung einer höheren Atemstabilität bzw. zur Erzielung einer Kennzahl, welche für eine höhere Atemstabilität steht, den Atemgasdruck und/oder den Atemgasfluss anzupassen. Alternativ oder zusätzlich kann die Auswerteeinrichtung dazu auch wenigstens einen der folgenden Parameter anpassen: inspiratorisches Druckniveau; exspiratorisches Druckniveau; Triggersensitivität; Inspirationsdauer; Exspirationsdauer; Steilheit der Druckübergänge zwischen den Druckniveaus.

Die Auswerteeinrichtung ist insbesondere dazu geeignet und ausgebildet, die Kennzahl erst ab einer definierten Mindestdauer der Beatmung zu ermitteln und/oder auszugeben und/oder für eine Aktion zu berücksichtigen. Insbesondere ist die Auswerteeinrichtung dazu geeignet und ausgebildet, die Kennzahl zusammen mit wenigstens einem Warnhinweis auf ein Unterschreiten der Mindestdauer auszugeben und/oder zusammen mit wenigstens einem Warnhinweis auf ein Unterschreiten der Mindestdauer für eine Aktion zu berücksichtigen.

Insbesondere umfasst die Mindestdauer wenigstens zwei Stunden und vorzugsweise wenigstens vier Stunden und besonders bevorzugt wenigstens sechs Stunden. Die Mindestdauer kann auch wenigstens acht Stunden oder wenigstens zehn Stunden oder auch 12 Stunden oder mehr betragen. Möglich ist auch eine Mindestdauer von einer halben Stunde oder einer Stunde oder eineinhalb Stunden. Besonders bevorzugt umfasst die Mindestdauer wenigstens eine Nachtschlafphase des Patienten. Dabei umfasst die Nachtschlafphase vorzugsweise wenigstens eine NREM-Phase und/oder REM-Phase. Insbesondere beträgt die Nachtschlafphase wenigstens eine Stunde und vorzugsweise wenigstens zwei Stunden und besonders bevorzugt wenigstens vier Stunden oder mehr.

In einer in einer vorteilhaften Ausgestaltung ist die Auswerteeinrichtung dazu geeignet und ausgebildet, eine akute Exazerbation zu erkennen, wenn die Kennzahl einen Anstieg der Atemstabilität des Patienten, insbesondere oberhalb einer definierten Steigungsrate, anzeigt und der erfasste Beatmungsparameter zugleich eine steigende Atemfrequenz und eine flachere Atmung anzeigt. Insbesondere wird als Folge einer solchen Erkennung wenigstens eine Aktion ausgeführt und beispielsweise ein Warnhinweis und/oder ein Alarm ausgegeben und/oder eine Handlungsanweisung ausgegeben. Insbesondere werden zur Erkennung eines solchen Ereignisses wenigstens zwei oder mehr Beatmungsparameter berücksichtigt und ausgewertet.

Das Auswertesystem kann wenigstens ein Beatmungsgerät umfassen. Das Beatmungsgerät ist insbesondere dazu geeignet und ausgebildet, in der zuvor beschriebenen Art und Weise betrieben und insbesondere von der Auswerteeinrichtung angesteuert zu werden. Insbesondere ist das Beatmungsgerät für eine Beatmung in der Art von CPAP und/oder APAP ausgebildet. Insbesondere ist die Auswerteeinrichtung dazu geeignet und ausgebildet, die Kennzahl während einer CPAP und/oder APAP und/oder einer anderen maschinellen Beatmung zu ermitteln. Insbesondere ist das Beatmungsgerät dazu geeignet und ausgebildet, mittels wenigstens einer Beatmungseinrichtung wenigstens eine definierte Atemgasströmung zur Beatmung zu erzeugen.

Insbesondere umfasst das Auswertesystem wenigstens ein

Sensormittel. Das Sensormittel kann von dem Beatmungsgerät bereitgestellt werden. Insbesondere weist das Beatmungsgerät eine Sensoreinrichtung auf, welche zur Steuerung bzw. Regelung der Beatmung dient. Beispielsweise ist wenigstens ein Flusssensor (Flowsensor) und/oder Drucksensor vorgesehen. Das Auswertesystem kann weitere Sensoren zur Erfassung von Beatmungsparametern aufweisen.

Das erfindungsgemäße Verfahren dient zum Betreiben eines Auswertesystems, wie es zuvor beschrieben wurde. Auch ein solches Verfahren löst die zuvor gestellte Aufgabe besonders vorteilhaft.

Insbesondere ist das Verfahren so ausgebildet, dass die zuvor beschriebenen Aktionen der Auswerteeinrichtung ausgeführt werden. Insbesondere ist das zuvor beschriebene Auswertesystem dazu geeignet und ausgebildet, nach dem erfindungsgemäßen Verfahren betrieben zu werden. Im Rahmen der vorliegenden Erfindung ist insbesondere vorgesehen, dass die Auswerteeinrichtung zur Ausführung der hier beschriebenen Verfahrensmerkmale bzw. Schritte geeignet und ausgebildet ist.

In der Auswerteeinrichtung ist insbesondere wenigstens ein Algorithmus hinterlegt, mittels dem aus einem oder mehreren Beatmungsparametern die Kennzahl berechnet werden kann. Insbesondere umfasst die Auswerteeinrichtung wenigstens eine elektronische Rechnereinheit.

Möglich ist, dass das Auswertesystem separat zu dem Beatmungsgerät ausgebildet ist. Beispielsweise ist die Auswerteeinrichtung dazu in einem vom Beatmungsgerät separaten Gehäuse untergebracht und kann mit diesem über wenigstens eine Schnittstelle kabelgebunden und/oder drahtlos gekoppelt werden.

Das Auswertesystem kann wenigstens eine (eigene) Anzeigeeinrichtung umfassen und/oder eine Anzeigeeinrichtung des Beatmungsgerätes nutzen, um die Kennzahl und/oder andere Informationen auszugeben. Die Anzeigeeinrichtung des Auswertesystems kann durch die Anzeigeeinrichtung des Beatmungsgerätes ergänzt werden. Das Auswertesystem kann auch mit wenigstens einem Rechner bzw. Computer und/oder wenigstens einem mobilen Endgerät oder dergleichen koppelbar sein, um dort die Kennzahl und/oder andere Informationen auszugeben. Dabei kann das Auswertesystem ein solches Gerät als eine Anzeigeeinrichtung nutzen.

Insbesondere wird der wenigstens eine Beatmungsparameter während der Beatmung erfasst. Vorzugsweise werden wenigstens zwei oder wenigstens drei und insbesondere eine Vielzahl von Beatmungsparametern während der Beatmung mittels des Sensormittels erfasst. Möglich ist auch, dass weitere Beatmungsparameter durch externe Sensoreinheiten erfasst und über die Zeit registriert und insbesondere zur Bestimmung der Kennzahl herangezogen werden. Solche weiteren Beatmungsparametern können beispielsweise Pulsfrequenz, Sauerstoffgehalt des Blutes, CO2-Gehalt des Blutes, Blutdruck, Körpertemperatur, Aktivität und/oder weitere Parameter sein. Wenn z. B. der Patient aktiv ist und sein mobiles Beatmungsgerät am Rollstuhl etc. nutzt, wird in dieser Phase eine hohe Variabilität anders bewertet als in der Nacht bei körperlicher Ruhe.

Der mittels des Sensormittels erfasste Beatmungsparameter kann aus wenigstens einer Sensorgröße direkt und/oder mittelbar abgeleitet sein. In allen Ausgestaltungen ist es bevorzugt, dass wenigstens zwei oder wenigstens drei und vorzugsweise eine Vielzahl von Beatmungsparametern erfasst und für die Ermittlung der Kennzahl berücksichtigt werden.

Weitere Vorteile und Merkmale der vorliegenden Erfindung ergeben sich aus der Beschreibung der Ausführungsbeispiele, welche mit Bezug auf die beiliegenden Figuren im Folgenden erläutert werden.

In den Figuren zeigen:
- Fig. 1: eine rein schematische Darstellung eines erfindungsgemäßen Auswertesystems in einer perspektivischen Ansicht;
- Fig. 2: ein stark schematisiertes Schaubild mit Verläufen von Beatmungsparametern zur Erläuterung der Ermittlung einer Kennzahl bei der Erfindung; und
- Fig. 3-7: weitere stark schematisierte Schaubilder mit Verläufen von Beatmungsparametern zur Erläuterung der Ermittlung der Kennzahl bei der Erfindung.

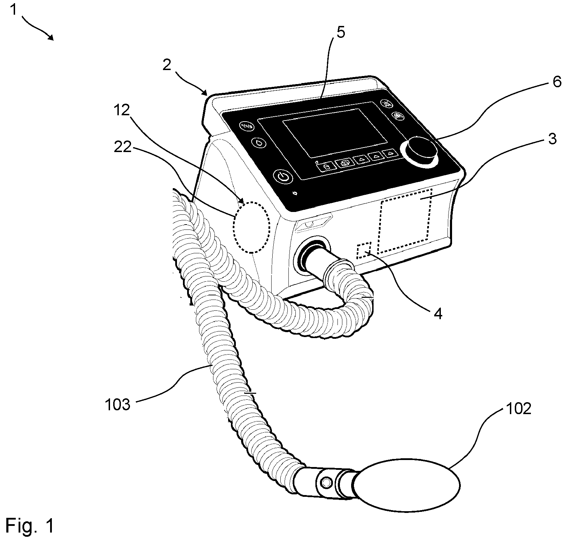

Die Figur 1 zeigt ein erfindungsgemäßes Auswertesystem 1 mit einer Auswerteeinrichtung 3 zur Bewertung der Atemstabilität während einer maschinellen Beatmung mit einem Beatmungsgerät 2. Das Beatmungsgerät 2 ist hier Teil des Auswertesystems 1 und stellt die Einhausung der Auswerteeinrichtung 3 bereit. Die Auswerteeinrichtung 3 kann alternativ auch als separate Vorrichtung außerhalb des Beatmungsgeräts 2 angeordnet sein. Das hier gezeigte System 1 wird nach dem erfindungsgemäßen Verfahren betrieben. Das Auswertesystem 1 ist hier als ein Heimbeatmungsgerät ausgebildet. Das Auswertesystem 1 kann aber auch zur klinischen Beatmung eingesetzt werden.

Das Beatmungsgerät 2 weist im Inneren seines Gehäuses hier eine Beatmungseinrichtung 12 auf, welche mit einer Lüftereinrichtung 22 zur Erzeugung einer Atemgasströmung ausgestattet ist. Die Atemgasströmung wird über eine an die Beatmungseinrichtung 12 gekoppelte Schlaucheinrichtung 103 mit einer Atemmaske 102 dem Patienten zugeführt. Alternativ zur Atemmaske 102 können auch andere Patientenschnittstellen verwendet werden. Zusätzlich oder alternativ zur Lüftereinrichtung 22 kann auch eine Druckgasquelle vorgesehen sein.

Das Beatmungsgerät 2 umfasst hier eine Anzeigeeinrichtung 5 und eine Bedieneinrichtung 6. Dabei können auch Kombinationen von Bedieneinrichtung 6 und Anzeigeeinrichtung 5 vorgesehen sein, beispielsweise in der Art einer berührungsempfindlichen Anzeigefläche bzw. eines Touchscreens. Die Anzeigeeinrichtung 5 dient hier auch zur Darstellung von Informationen der Auswerteeinrichtung 3. Die Auswerteeinrichtung 3 kann ihre Informationen aber auch auf weiteren hier nicht gezeigten Anzeigeeinrichtungen ausgeben, z. B. auf einem Computerdisplay oder einem Tablet oder Smartphone oder dergleichen.

Die Beatmungseinrichtung 12 ist hier mit einem Sensormittel 4 wirkverbunden, welches ein oder mehrere Sensoren zur Erfassung von Beatmungsparametern und ggf. weiterer für die Beatmung charakteristischer Größen aufweist. Zum Beispiel umfasst das Sensormittel 4 einen hier nicht näher gezeigten Drucksensor, welcher die Druckverhältnisse der Atemgasströmung erfasst. Das Sensormittel 4 ist hier auch mit der Auswerteeinrichtung 3 wirkverbunden, sodass die erfassten Größen wenigstens teilweise auch von der Auswerteeinrichtung 3 verarbeitet werden können. Die Auswerteeinrichtung 3 kann auch eigene Sensormittel 4 umfassen.

Die Beatmungseinrichtung 12 umfasst hier eine nicht sichtbar innerhalb des Gehäuses angeordnete Steuereinrichtung zur Ansteuerung der Lüftereinrichtung 22. So können z. B. eine CPAP-Beatmung oder eine APAP-Beatmung ausgeführt werden. Für eine Beatmung wird die Beatmungseinrichtung 12 z. B. auf einen definierten Atemgasfluss und/oder ein Atemgasdruck eingestellt. Die Steuereinrichtung 12 kann einen notwendigen Minimaldruck bereitstellen und/oder Druckschwankungen kompensieren, die durch die Atemtätigkeit des Benutzers bedingt sind. Beispielsweise erfasst die Steuereinrichtung 12 mittels des Sensormittels 4 den gegenwärtigen Druck in der Atemmaske 102 und regelt die Leistung der Lüftereinrichtung 22 entsprechend nach, bis ein gewünschter Beatmungsdruck anliegt.

Zudem kann die Beatmungseinrichtung 12 hier auch von der Auswerteeinrichtung 3 angesteuert werden. Dazu ist die Auswerteeinrichtung 3 mit der Steuereinrichtung wirkverbunden.

Um eine Bewertung der Qualität der Beatmung vorzunehmen, erfasst die Auswerteeinrichtung 3 während der Beatmung mittels des Sensormittels 4 mehrere Beatmungsparameter und registriert diese über die Zeit. Aus dem zeitlichen Verlauf der Beatmungsparameter wird dann eine Kennzahl für eine Atemstabilität des Patienten ermittelt und auf der Anzeigeeinrichtung 5 dargestellt. So erhält der Benutzer durch einen Blick auf die Kennzahl eine Bewertung der Qualität der Beatmung.

Die Berechnung der Atemstabilität erfolgt hier durch statistische Auswertung des Atemmusters (z. B. im CPAP- oder APAP-Modus). Bei der Beatmung kann stark in das Atemmuster eingegriffen werden. Idealerweise wäre bei einer mechanischen Beatmung das Atemmuster komplett starr. Bei einer assistierten Beatmung ist eine gewisse Variabilität durch den Patienten möglich und gewünscht. Im Laufe einer Beatmung über einen längeren Zeitraum, vorzugsweise mindestens einen Teil des Nachtschlafs beinhaltend, sollten jedoch bei einer guten Beatmungseinstellung hinreichend lange Phasen mit stabiler Atmung erreicht werden. Ist dies nicht der Fall, so spricht dies für eine nicht optimal eingestellte Beatmung.

Der berechnete Indikator, vorzugsweise zwischen 0 und 100, für die Atemstabilität dient also als Hinweis / Basis für einen Alarm für einen Optimierungsbedarf der Beatmung oder eine akute Verschlechterung des Zustandes des Patienten. Eine durchweg niedrige Atemstabilität kann verursacht werden z. B. durch: Asynchronität zwischen Beatmungsgerät (2) und Patient; Auftreten von partiellen oder vollständigen intermittierenden Atemwegsobstruktionen; Insomnie; Beschwerden durch Maske (102) und/oder Therapiedruck; Atemnot; starke Sekretbildung; Hustenreiz; instabilen Atemantrieb; periodische Atmung; starke Leckagen.

Der Wert der Kennzahl bzw. des Atemstabilitäts-Indikators ermöglicht eine rasche Orientierung, ob eine weitergehende Analyse des Patienten erforderlich ist. In Kombination mit weiteren gemessenen Parametern wie AHI oder Asynchronie-Ereignisse können einzelne Ursachen für eine instabile Atmung entweder bestätigt oder ausgeschlossen werden. Bevorzugt ist es dem System 1 dadurch möglich, Beatmungseinstellungen wie inspiratorisches Druckniveau, exspiratorisches Druckniveau, Triggersensitivität, Inspirationsdauer, Exspirationsdauer oder Steilheit der Druckübergänge zwischen den Druckniveaus automatisch anzupassen und den Erfolg über eine Verbesserung der Atemstabilität zu messen. Außerdem kann eine Warnung für eine akute Verschlechterung des Krankheitszustandes / Exazerbation ausgegeben werden, bevorzugt in Kombination mit weiteren Parametern wie Atemfrequenz oder Atemtiefe (Tidalvolumen bzw. Quotient aus Frequenz und Tidalvolumen). Mögliche Regel: Steigende Frequenz, flachere Atmung und starrere Atmung (Anstieg der Atemstabilität) können ein Indikator für eine akute Exazerbation sein.

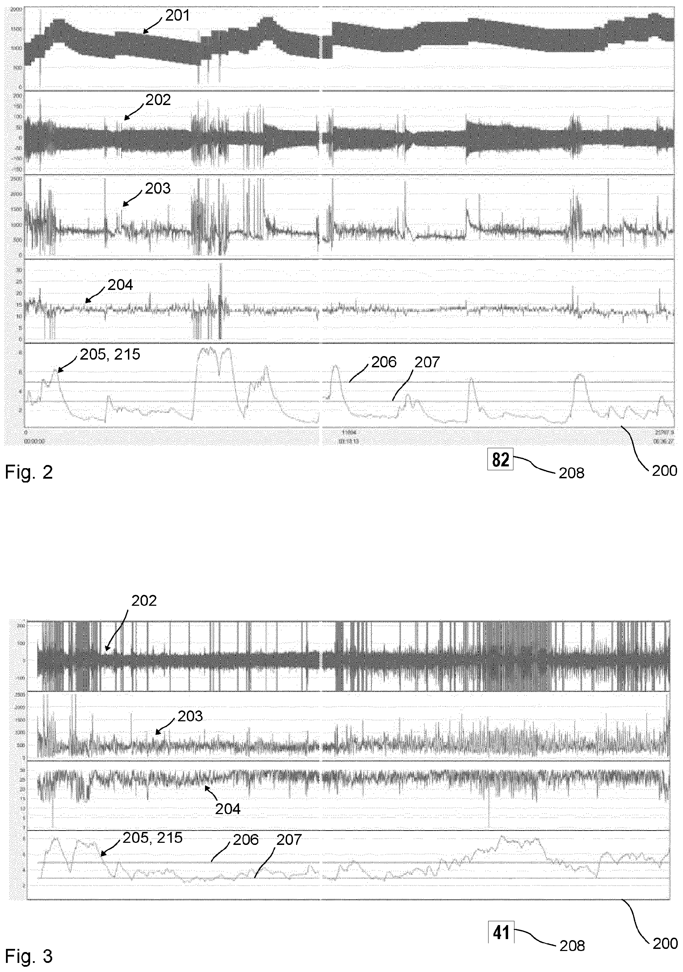

Die Figur 2 zeigt eine beispielhafte Ermittlung und Ausgabe einer Kennzahl 208 sowie die ihrer Berechnung zugrunde liegenden Beatmungsparameter. Die Beatmungsparameter wurden hier durch die Sensormittel 4 während der Beatmung erfasst und als Verläufe über die Zeit 200 registriert. Der Zeitraum der Messung beträgt hier 6:30 h.

Die Beatmungsparameter sind hier der Atemgasdruck 201, der Atemgasfluss bzw. Flow 202 und das Atemvolumen bzw. Atemzugvolumen 203 sowie die Atemfrequenz 204. Aus deren Verläufen wurde eine Stabilitätsrate 205 ermittelt, welche in dem hier gezeigten Beispiel als eine Variabilitätsrate 215 ausgebildet ist und die Atemvariabilität beschreibt. Die Variabilitätsrate 215 nimmt also einen höheren Wert an, wenn die Atmung variabler bzw. instabiler wird und umgekehrt. Eine solche Variabilitätsrate 215 kann z. B. dem Kehrwert der gewünschten Kennzahl 208 entsprechen.

Ist der Wert der Variabilitätsrate 215 hoch, schwankt der Atemfluss / das Atemvolumen / die Atemfrequenz stark zwischen einzelnen Atemzügen. Ist der Wert niedrig, so ist die Atmung gleichmäßig.

Die beiden Linien entlang der Stabilitätsrate 205 bzw. der Variabilitätsrate 215 markieren hier einen Schwellwert 207 für die Stabilität und einen Schwellwert 206 für die Instabilität. Zeiträume, bei denen die Kurve 215 unterhalb der unteren Linie 207 liegt, werden als vollständig stabil gewertet. Die Kennzahl 208 erhält dann den maximal zur Bewertung vorgesehenen Wert (z. B. Score 100).

Zeiträume, bei denen die Kurve 215 oberhalb der oberen Linie 206 liegt, werden als vollständig instabil gewertet. Die Kennzahl 208 erhält dann den minimal zur Bewertung vorgesehenen Wert (z. B. Score 0).

Zwischen den beiden Schwellenwerten 206, 207 wird hier linear interpoliert zwischen 0 und 100. So ergibt sich für die hier beispielhaft gezeigte Beatmung des Patienten als Kennzahl 208 ein mittlerer Stabilitäts-Score von 82. Das ist ein guter Wert, die Atmung ist über lange Phasen gleichmäßig.

Beispielsweise kann zur Bestimmung der Stabilitätsrate 205 bzw. Variabilitätsrate 215 aus erfassten Beatmungsparametern die folgende Formel in der Auswerteinrichtung 3 hinterlegt sein und eingesetzt werden: "Variabilitätsrate = Gleitender_ Mittelwert_über_2min (Betrag ((Beatmungs_Parameter_aktueller_Atemzug - Beatmungs_Parameter_Mittelwert_über_2min)/ Beatmungs_Parameter_Mittelwert_über_2min) *100)".

Beispielsweise kann dann zur Herleitung der Kennzahl 208 aus der Stabilitätsrate 205 bzw. Variabilitätsrate 215 die folgende Formel in der Auswerteinrichtung 3 hinterlegt sein und eingesetzt werden: "Kennzahl_gesamte_Messung = 100-Mittelwert_gesamte_Messung (Variabilitätsrate)".

Die Auswerteinrichtung 3 kann hier so ausgebildet sein, dass für zwei oder mehr Beatmungsparameter unterschiedliche Stabilitätsraten 205 bzw. Variabilitätsraten 215 berechnet und verglichen werden. Schwankt z. B. in einer Phase die Atemfrequenz stärker als das Tidalvolumen, so deutet es auf Asynchronie hin. Schwankt das Volumen stärker als die Frequenz, deutet dies auf Apnoen oder Flusslimitationen hin.

Die Figur 3 zeigt eine weitere beispielhafte Ermittlung und Ausgabe einer Kennzahl 208 sowie die ihrer Berechnung zugrunde liegenden Beatmungsparameter. Der Patient weist hier viele Apnoen / Atemaussetzer auf. Diese werden hier durch die senkrechten Linien im Atemfluss 202 skizziert. Die Atmung ist hier so gut wie nie richtig stabil. Für die hier beispielhaft gezeigte Beatmung des Patienten ergibt sich als Kennzahl 208 ein mittlerer Score von 41.

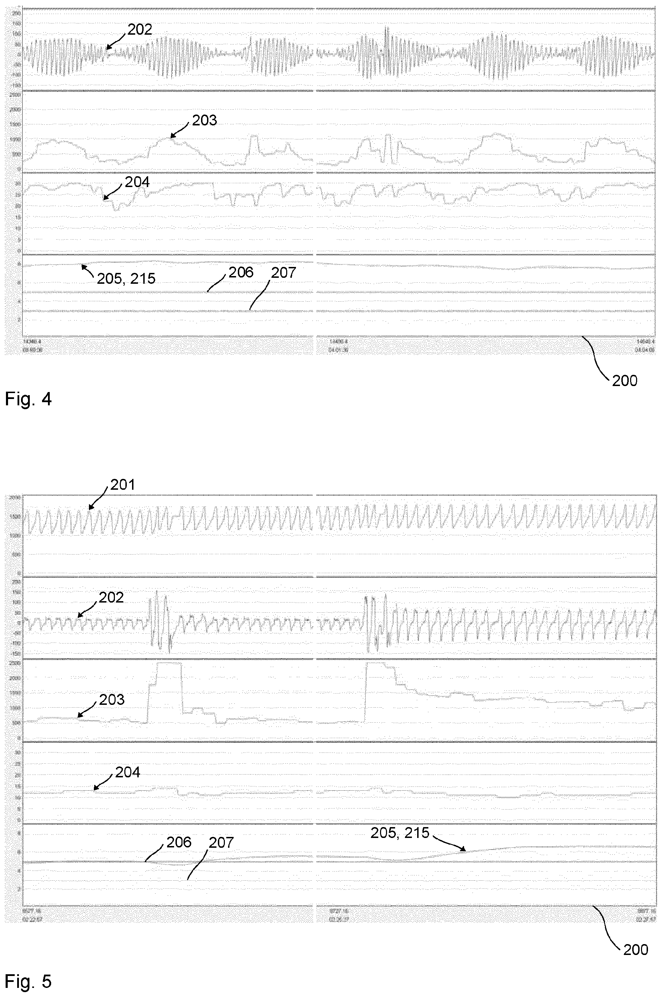

Die Figuren 4 bis 7 zeigen Beispiele von Situationen, die zu Atem-Unruhe führen können. Daher zeigen sich hier signifikant erhöhte Variabilitätsraten 215. Die Variabilitätsraten 215 beschreiben hier jeweils, wie auch in den Figuren 2 und 3 zuvor, die Atemvariabilität.

Die Figur 4 zeigt ein Atemmuster mit Apnoen.

Die Figur 5 zeigt ein Atemmuster mit Flusslimitationen, welche durch Arousals unterbrochen werden. Die Flusslimitationen können auf einem erhöhten Widerstand der oberen Atemwege beruhen.

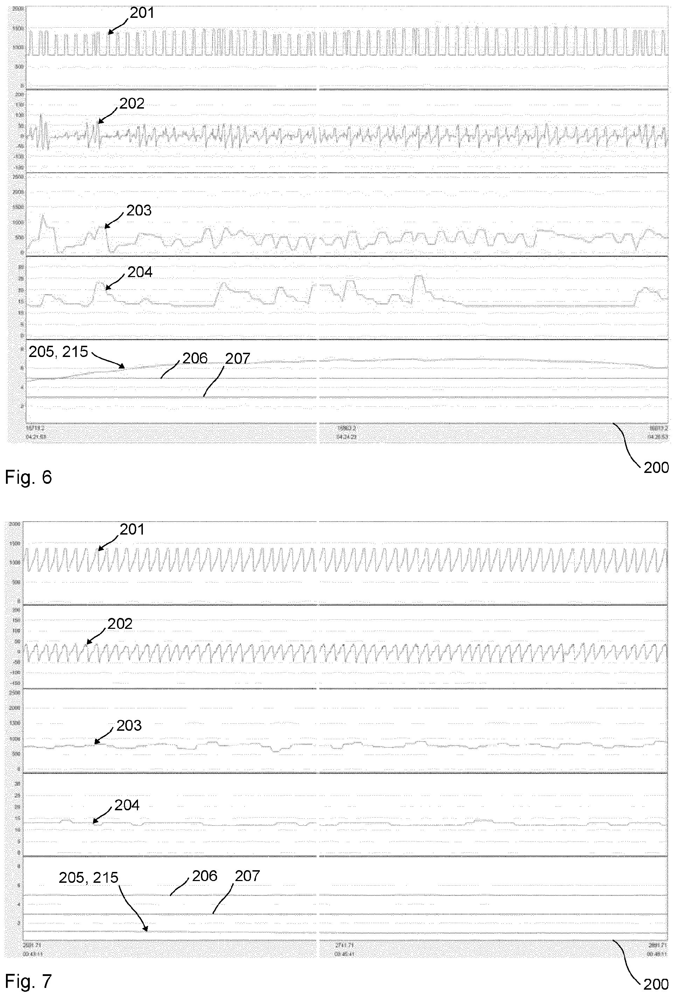

Die Figur 6 zeigt ein Atemmuster mit einer erheblichen Asynchronität, sodass hier das Gerät 1 und der Patient gegeneinander arbeiten.

Die Figur 6 zeigt ein Atemmuster mit einer besonders gleichmäßigen Atmung. Die Stabilitätsrate 205, hier als Variabilitätsrate 215 ausgebildet, liegt hier dauerhaft unterhalb des Schwellenwerts 207, sodass die Kennzahl 208 hier einen maximal möglichen Wert annimmt.

### Bezugszeichenliste:

- 1: Auswertesystem
- 2: Beatmungsgerät
- 3: Auswerteeinrichtung
- 4: Sensormittel
- 5: Anzeigeeinrichtung
- 6: Bedieneinrichtung
- 12: Beatmungseinrichtung
- 22: Lüftereinrichtung
- 102: Atemmaske
- 103: Schlaucheinrichtung
- 200: Zeit
- 201: Druck
- 202: Fluss
- 203: Volumen
- 204: Frequenz
- 205: Stabilitätsrate
- 206: Schwellenwert
- 207: Schwellenwert
- 208: Kennzahl
- 215: Variabilitätsrate

## Patentansprüche

1. Auswertesystem (1) für wenigstens ein zum maschinellen Beatmen eines Patienten vorgesehenes Beatmungsgerät (2), umfassend wenigstens eine Auswerteeinrichtung (3), welche dazu geeignet und ausgebildet ist, wenigstens zwei unterschiedliche mittels wenigstens eines Sensormittels (4) erfassten Beatmungsparameter über die Zeit zu registrieren, wobei die zwei unterschiedlichen Beatmungsparameter ausgewählt sind aus der Gruppe Atemfrequenz, Atemvolumen, Atemgasfluss, Atemgasdruck, wobei
die Auswerteeinrichtung (3) dazu geeignet und ausgebildet ist, aus dem zeitlichen Verlauf der wenigstens zwei unterschiedlichen Beatmungsparameter wenigstens eine Stabilitätsrate zu berechnen und aus der Stabilitätsrate wenigstens eine Kennzahl für eine Atemstabilität des Patienten zu ermitteln, sodass die Kennzahl eine Bewertung der Qualität der Beatmung während eines Zeitraums bereitstellt,
wobei die Auswerteeinrichtung (3) dazu geeignet und ausgebildet ist, die Kennzahl während der Beatmung zu ermitteln und in bestimmten Zeitabständen und fortlaufend zu aktualisieren, **dadurch gekennzeichnet, dass**
die Kennzahl zurückliegende Zeiträume und die diesen Zeiträumen zugeordneten Werte für die Kennzahl wenigstens teilweise berücksichtigt.

2. Auswertesystem (1) nach dem vorhergehenden Anspruch, wobei die Auswerteeinrichtung (3) dazu geeignet und ausgebildet ist, die Kennzahl als wenigstens eine Zahl und/oder Grafik und/oder wenigstens ein Symbol und/oder Wort auszugeben und vorzugsweise auf wenigstens einer Anzeigeeinrichtung (5) anzuzeigen.

3. Auswertesystem (1) nach einem der vorhergehenden Ansprüche, wobei die ermittelte Kennzahl wenigstens einem Wert auf einer Skala entspricht und wobei einem Ende der Skala eine höhere bzw. maximale Atemstabilität und einem anderen Ende der Skala eine geringere bzw. minimale Atemstabilität zugeordnet ist wobei die Auswerteeinrichtung (3) dazu geeignet und ausgebildet ist, den Wert und/oder die Skala mit dem darauf positionierten Wert auszugeben und vorzugsweise auf wenigstens einer Anzeigeeinrichtung (5) anzuzeigen.

4. Auswertesystem (1) nach Anspruch 1, wobei die Auswerteeinrichtung (3) dazu geeignet und ausgebildet ist, einem Zeitraum, in welchem die Stabilitätsrate einen Schwellenwert (207) für die Stabilität überschreitet, eine Kennzahl mit einem für eine maximale Atemstabilität vorgesehenen Wert zuzuordnen und/oder einem Zeitraum, in welchem die Stabilitätsrate einen Schwellenwert (206) für die Instabilität überschreitet, eine Kennzahl mit einem für eine minimale Atemstabilität vorgesehenen Wert zuzuordnen.

5. Auswertesystem (1) nach dem vorhergehenden Anspruch, wobei die Auswerteeinrichtung (3) dazu geeignet und ausgebildet ist, einem Zeitraum, in welchem die Stabilitätsrate zwischen dem Schwellenwert für die Stabilität und dem Schwellenwert für die Instabilität liegt, eine Kennzahl mit einem Wert zuzuordnen und den Wert mittels wenigstens einer Zuordnungsfunktion und vorzugsweise mittels linearer Interpolation aus der Stabilitätsrate zu berechnen.

6. Auswertesystem (1) nach einem der vorhergehenden Ansprüche, wobei die Auswerteeinrichtung (3) dazu geeignet und ausgebildet ist, zur Ermittlung der Kennzahl wenigstens einen statistischen Kennwert für eine Tendenz des zeitlichen Verlaufs des Beatmungsparameters heranzuziehen und wobei der statistische Kennwert vorzugsweise ein Mittelwert und besonders bevorzugt ein gleitender Mittelwert ist oder wenigstens einen solchen umfasst.

7. Auswertesystem (1) nach einem der vorhergehenden Ansprüche, wobei die Auswerteeinrichtung (3) dazu geeignet und ausgebildet ist, die Kennzahl wenigstens aus wenigstens einem statistischen Streuungsmaß für eine Verteilung von Werten im zeitlichen Verlauf des Beatmungsparameters zu ermitteln und wobei das statistische Streuungsmaß vorzugsweise eine Varianz ist oder wenigstens eine solche umfasst.

8. Auswertesystem (1) nach einem der vorhergehenden Ansprüche, wobei die Auswerteeinrichtung (3) dazu geeignet und ausgebildet ist, eine Tendenz für eine erfolgte und/oder zukünftige Entwicklung der Kennzahl zu berechnen und auszugeben.

9. Auswertesystem (1) nach einem der vorhergehenden Ansprüche, wobei die Auswerteeinrichtung (3) dazu geeignet und ausgebildet ist, aufgrund wenigstens einer der folgenden Ursachen die Kennzahl in Richtung schlechter Atemstabilität zu verändern: Asynchronität zwischen Beatmungsgerät (2) und Patient; Auftreten von partiellen oder vollständigen intermittierenden Atemwegsobstruktionen; Insomnie; Beschwerden durch Maske (102) und/oder Therapiedruck; Atemnot; starke Sekretbildung; Hustenreiz; instabilen Atemantrieb; periodische Atmung; starke Leckagen.

10. Auswertesystem (1) nach einem der vorhergehenden Ansprüche, wobei die Auswerteeinrichtung (3) dazu geeignet und ausgebildet ist, wenigstens eine Aktion auszuführen, wenn die Kennzahl in wenigstens einem definierten kritischen Wertebereich für die Atemstabilität liegt und/oder eine Tendenz dahin aufweist und wobei die Aktion eine Ausgabe wenigstens eines Warnhinweises und/oder Alarms und/oder einer Handlungsbeschreibung umfasst.

11. Auswertesystem (1) nach dem vorhergehenden Anspruch, wobei sowohl wenigstens ein kritischer Wertebereich für eine zu hohe als auch für eine zu niedrige Atemstabilität vorgesehen ist, sodass auch eine zu starre Atmung zu einer Aktion führen kann.

12. Auswertesystem (1) nach einem der vorhergehenden Ansprüche, wobei die Auswerteeinrichtung (3) dazu geeignet und ausgebildet ist, die Kennzahl in Kombination mit wenigstens einem Wert für wenigstens einen Beatmungsparameter auszugeben, beispielsweise Atemfrequenz und/oder Atemtiefe, wobei der Wert vorzugsweise statistisch über die Zeit aufbereitet ist.

13. Auswertesystem (1) nach einem der vorhergehenden Ansprüche, wobei die Auswerteeinrichtung (3) dazu geeignet und ausgebildet ist, das Beatmungsgerät (2) in Abhängigkeit der ermittelten Kennzahl anzusteuern und insbesondere wenigstens eine Steuergröße des Beatmungsgeräts (2) anzupassen, um eine Veränderung der Kennzahl in Richtung einer höheren Atemstabilität zu erreichen.

14. Auswertesystem (1) nach dem vorhergehenden Anspruch, wobei die Auswerteeinrichtung (3) dazu geeignet und ausgebildet ist, zur Erzielung einer höheren Atemstabilität den Atemgasdruck und/oder den Atemgasfluss anzupassen und/oder wenigstens einen der folgenden Parameter anzupassen:
inspiratorisches Druckniveau; exspiratorisches Druckniveau; Triggersensitivität; Inspirationsdauer; Exspirationsdauer; Steilheit der Druckübergänge zwischen den Druckniveaus.

15. Auswertesystem (1) nach einem der vorhergehenden Ansprüche, wobei die Auswerteeinrichtung (3) dazu geeignet und ausgebildet ist, die Kennzahl erst ab einer definierten Mindestdauer der Beatmung auszugeben und/oder für eine Aktion zu berücksichtigen und/oder wobei die Auswerteeinrichtung (3) dazu geeignet und ausgebildet ist, die Kennzahl zusammen mit wenigstens einem Warnhinweis für ein Unterschreiten der Mindestdauer auszugeben und/oder zusammen mit wenigstens einem Warnhinweis für ein Unterschreiten der Mindestdauer für eine Aktion zu berücksichtigen, wobei die Mindestdauer wenigstens zwei Stunden und/oder wenigstens eine Nachtschlafphase des Patienten umfasst.

16. Auswertesystem (1) nach einem der vorhergehenden Ansprüche, wobei die Auswerteeinrichtung (3) dazu geeignet und ausgebildet ist, eine akute Exazerbation zu erkennen, wenn die Kennzahl einen Anstieg der Atemstabilität des Patienten anzeigt und der erfasste Beatmungsparameter zugleich eine steigende Atemfrequenz und eine flachere Atmung anzeigt.

17. Auswertesystem (1) nach einem der vorhergehenden Ansprüche, umfassend wenigstens ein Beatmungsgerät (2).

18. Verfahren zum Betreiben eines Auswertesystems (1) nach Anspruch 1.

## Claims

1. An evaluation system (1) for at least one ventilator (2) intended for mechanically ventilating a patient, comprising at least one evaluation apparatus (3) which is suitable and designed for recording over time at least two different ventilation parameters detected by means of at least one sensor means (4), wherein the two different ventilation parameters are selected from the group consisting of respiratory rate, breathing volume, breathing gas flow, breathing gas pressure, wherein the evaluation apparatus (3) is suitable and designed for calculating at least one stability rate from the time course of the at least two different ventilation parameters and for determining at least one index for a respiratory stability of the patient from the stability rate, such that the index provides an assessment of the quality of the ventilation over a time period,
wherein the evaluation apparatus (3) is suitable and designed for determining the index during ventilation and for updating it at specific time intervals and continuously, **characterized in that** the index at least partially takes into account past time periods and the values for the index assigned to those time periods.

2. The evaluation system (1) according to the preceding claim, wherein the evaluation apparatus (3) is suitable and designed for outputting the index as at least one number and/or graphic and/or at least one symbol and/or word and preferably for displaying it on at least one display apparatus (5).

3. The evaluation system (1) according to one of the preceding claims, wherein the determined index corresponds to at least one value on a scale, and wherein one end of the scale is associated with a higher or rather maximum respiratory stability and the other end of the scale is associated with a lower or rather minimum respiratory stability, wherein the evaluation apparatus (3) is suitable and designed for outputting the value and/or the scale with the value positioned thereon and preferably for displaying same on at least one display apparatus (5).

4. The evaluation system (1) according to claim 1, wherein the evaluation apparatus (3) is suitable and designed for assigning, to a time period in which the stability rate exceeds a threshold value (207) for stability, an index having a value provided for a maximum respiratory stability and/or for assigning, to a time period in which the stability rate exceeds a threshold value (206) for instability, an index having a value provided for a minimum respiratory stability.

5. The evaluation system (1) according to the preceding claim, wherein the evaluation apparatus (3) is suitable and designed for assigning an index having a value to a time period in which the stability rate lies between the threshold value for stability and the threshold value for instability, and for calculating the value from the stability rate by means of at least one mapping function and preferably by means of linear interpolation.

6. The evaluation system (1) according to one of the preceding claims, wherein the evaluation apparatus (3) is suitable and designed for using at least one statistical characteristic value for a trend in the time course of the ventilation parameter in order to determine the index, and wherein the statistical characteristic value is preferably a mean value and, particularly preferably, a moving mean value, or at least comprises such a value.

7. The evaluation system (1) according to one of the preceding claims, wherein the evaluation apparatus (3) is suitable and designed for determining the index at least from at least one statistical measure of dispersion for a distribution of values over the time course of the ventilation parameter, and wherein the statistical measure of dispersion is preferably a variance or at least comprises such a variance.

8. The evaluation system (1) according to one of the preceding claims, wherein the evaluation apparatus (3) is suitable and designed for calculating and outputting a trend for a past and/or future development of the index.

9. The evaluation system (1) according to one of the preceding claims, wherein the evaluation apparatus (3) is suitable and designed for changing the index in the direction of poor respiratory stability on the basis of at least one of the following causes: asynchrony between the ventilator (2) and the patient; occurrence of partial or complete intermittent airway obstructions; insomnia; discomfort due to the mask (102) and/or treatment pressure; dyspnea; excessive secretion; urge to cough; unstable respiratory drive; periodic breathing; severe leaks.

10. The evaluation system (1) according to one of the preceding claims, wherein the evaluation apparatus (3) is suitable and designed for performing at least one action when the index lies in at least one defined critical value range for the respiratory stability and/or shows a trend toward that, and wherein the action comprises an output of at least one warning notice and/or alarm and/or an instruction for action.

11. The evaluation system (1) according to the preceding claim, wherein at least one critical value range is provided both for excessively high and for excessively low respiratory stability, such that excessively rigid breathing can also trigger an action.

12. The evaluation system (1) according to one of the preceding claims, wherein the evaluation apparatus (3) is suitable and designed for outputting the index in combination with at least one value for at least one ventilation parameter, for example respiratory rate and/or breathing depth, wherein the value is preferably statistically processed over time.

13. The evaluation system (1) according to one of the preceding claims, wherein the evaluation apparatus (3) is suitable and designed for controlling the ventilator (2) depending on the determined index and, in particular, for adjusting at least one control variable of the ventilator (2) in order to effect a change of the index in the direction of higher respiratory stability.

14. The evaluation system (1) according to the preceding claim, wherein the evaluation apparatus (3) is suitable and designed, in order to achieve a higher respiratory stability, for adjusting the breathing gas pressure and/or the breathing gas flow and/or for adjusting at least one of the following parameters: inspiratory pressure level; expiratory pressure level; trigger sensitivity; inspiration duration; expiration duration; slope of the pressure transitions between the pressure levels.

15. The evaluation system (1) according to one of the preceding claims, wherein the evaluation apparatus (3) is suitable and designed for outputting the index and/or for taking same into account for an action only after a defined minimum duration of the ventilation has elapsed, and/or wherein the evaluation apparatus (3) is suitable and designed for outputting the index together with at least one warning notice indicating that the minimum duration has not been reached and/or for taking the index into account for an action together with at least one warning notice indicating that the minimum duration has not been reached, wherein the minimum duration comprises at least two hours and/or at least one night sleep phase of the patient.

16. The evaluation system (1) according to one of the preceding claims, wherein the evaluation apparatus (3) is suitable and designed for identifying an acute exacerbation when the index indicates an increase of the respiratory stability of the patient and the detected ventilation parameter simultaneously indicates an increasing respiratory rate and shallower breathing.

17. The evaluation system (1) according to one of the preceding claims, comprising at least one ventilator (2).

18. A method for operating an evaluation system (1) according to claim 1.

## Revendications

1. Système d'évaluation (1) pour au moins un appareil respiratoire (2) destiné à ventiler mécaniquement un patient, comprenant au moins une unité d'évaluation (3) adaptée et configurée pour enregistrer au cours du temps au moins deux paramètres de ventilation distincts détectés au moyen d'au moins un moyen de capteur (4), les deux paramètres de ventilation distincts étant choisis dans le groupe constitué par la fréquence respiratoire, le volume respiratoire, le flux de gaz respiratoire, la pression de gaz respiratoire, l'unité d'évaluation (3) étant adaptée et configurée pour calculer au moins un taux de stabilité à partir de l'évolution temporelle des au moins deux paramètres de ventilation distincts, et pour déterminer au moins un indice de stabilité respiratoire du patient à partir du taux de stabilité, de sorte que l'indice fournit une évaluation de la qualité de la ventilation pendant une période,
l'unité d'évaluation (3) étant adaptée et configurée pour déterminer l'indice pendant la ventilation et pour le mettre à jour à des intervalles de temps déterminés et de manière continue, **caractérisé en ce que** l'indice prend au moins partiellement en compte des périodes passées et les valeurs de l'indice associées à ces périodes.

2. Système d'évaluation (1) selon la revendication précédente, dans lequel l'unité d'évaluation (3) est adaptée et configurée pour fournir l'indice sous forme d'au moins un nombre et/ou d'un graphique et/ou d'au moins un symbole et/ou d'un mot et, de préférence, pour l'afficher sur au moins un dispositif d'affichage (5).

3. Système d'évaluation (1) selon l'une des revendications précédentes, dans lequel l'indice déterminé correspond au moins à une valeur sur une échelle et dans lequel une extrémité de l'échelle est associée à une stabilité respiratoire plus élevée ou maximale et l'autre extrémité de l'échelle est associée à une stabilité respiratoire plus faible ou minimale, l'unité d'évaluation (3) étant adaptée et configurée pour fournir ladite valeur et/ou ladite échelle avec ladite valeur positionnée sur celle-ci et, de préférence, pour l'afficher sur au moins un dispositif d'affichage (5).

4. Système d'évaluation (1) selon la revendication 1, dans lequel l'unité d'évaluation (3) est adaptée et configurée pour associer, à une période pendant laquelle le taux de stabilité dépasse une valeur seuil (207) de stabilité, un indice ayant une valeur prévue pour une stabilité respiratoire maximale, et/ou pour associer, à une période pendant laquelle le taux de stabilité dépasse une valeur seuil (206) d'instabilité, un indice ayant une valeur prévue pour une stabilité respiratoire minimale.

5. Système d'évaluation (1) selon la revendication précédente, dans lequel l'unité d'évaluation (3) est adaptée et configurée pour associer, à une période dans laquelle le taux de stabilité se situe entre la valeur seuil de stabilité et la valeur seuil d'instabilité, un indice ayant une valeur, et pour calculer la valeur à partir du taux de stabilité au moyen d'au moins une fonction d'association et, de préférence, au moyen d'une interpolation linéaire.

6. Système d'évaluation (1) selon l'une des revendications précédentes, dans lequel, pour déterminer l'indice, l'unité d'évaluation (3) est adaptée et configurée pour prendre en compte au moins un indicateur statistique caractérisant une tendance de l'évolution temporelle du paramètre de ventilation, et dans lequel le paramètre statistique est ou du moins comporte de préférence une moyenne et, de façon particulièrement préférée, une moyenne mobile.

7. Système d'évaluation (1) selon l'une des revendications précédentes, dans lequel l'unité d'évaluation (3) est adaptée et configurée pour déterminer l'indice au moins à partir d'au moins une mesure statistique de dispersion pour une distribution de valeurs dans l'évolution temporelle du paramètre de ventilation, et dans lequel la mesure statistique de dispersion est ou du moins comporte de préférence une variance.

8. Système d'évaluation (1) selon l'une des revendications précédentes, dans lequel l'unité d'évaluation (3) est adaptée et configurée pour calculer et fournir une tendance passée et/ou future d'une évolution de l'indice.

9. Système d'évaluation (1) selon l'une des revendications précédentes, dans lequel l'unité d'évaluation (3) est adaptée et configurée pour modifier, en raison d'au moins l'une des causes suivantes, l'indice dans le sens d'une stabilité respiratoire réduite : asynchronisme entre l'appareil respiratoire (2) et le patient ; survenue d'obstructions intermittentes partielles ou complètes des voies aériennes ; insomnie ; gêne due au masque (102) et/ou à la pression thérapeutique ; dyspnée ; forte production de sécrétions ; toux irritative ; commande respiratoire instable ; respiration périodique ; fuites importantes.

10. Système d'évaluation (1) selon l'une des revendications précédentes, dans lequel l'unité d'évaluation (3) est adaptée et configurée pour exécuter au moins une action lorsque l'indice se situe dans au moins une plage de valeurs critique définie pour la stabilité respiratoire et/ou présente une tendance en ce sens, et dans lequel l'action comporte l'émission d'au moins un avertissement et/ou d'une alarme et/ou d'une consigne d'action.

11. Système d'évaluation (1) selon la revendication précédente, dans lequel il est prévu au moins une plage de valeurs critique aussi bien pour une stabilité respiratoire trop élevée que pour une stabilité respiratoire trop faible, de sorte qu'une respiration trop rigide peut également entraîner une action.

12. Système d'évaluation (1) selon l'une quelconque des revendications précédentes, dans lequel l'unité d'évaluation (3) est adaptée et configurée pour fournir l'indice en combinaison avec au moins une valeur pour au moins un paramètre de ventilation, par exemple une fréquence respiratoire et/ou une profondeur respiratoire, la valeur étant de préférence traitée statistiquement au cours du temps.

13. Système d'évaluation (1) selon l'une des revendications précédentes, l'unité d'évaluation (3) étant adaptée et configurée pour commander l'appareil respiratoire (2) en fonction de l'indice déterminé et, en particulier, pour ajuster au moins un paramètre de commande de l'appareil respiratoire (2) afin d'obtenir une modification de l'indice vers une stabilité respiratoire plus élevée.

14. Système d'évaluation (1) selon la revendication précédente, dans lequel, pour accroître la stabilité respiratoire, l'unité d'évaluation (3) est adaptée et configurée pour ajuster la pression du gaz respiratoire et/ou le flux de gaz respiratoire et/ou au moins l'un des paramètres suivants : niveau de pression inspiratoire ; niveau de pression expiratoire ; sensibilité de déclenchement ; durée d'inspiration ; durée d'expiration ; pente des transitions de pression entre les niveaux de pression.

15. Système d'évaluation (1) selon l'une des revendications précédentes, dans lequel l'unité d'évaluation (3) est adaptée et configurée pour ne fournir l'indice et/ou le prendre en compte en vue d'une action seulement à partir d'une durée minimale définie de ventilation et/ou dans lequel l'unité d'évaluation (3) est adaptée et configurée pour fournir l'indice conjointement avec au moins un avertissement de non-respect de la durée minimale et/ou pour le prendre en compte en vue d'une action conjointement avec au moins un avertissement de non-respect de la durée minimale, dans lequel la durée minimale comporte au moins deux heures et/ou au moins une phase de sommeil nocturne du patient.

16. Système d'évaluation (1) selon l'une des revendications précédentes, dans lequel l'unité d'évaluation (3) est adaptée et configurée pour détecter une exacerbation aiguë lorsque l'indice indique une augmentation de la stabilité respiratoire du patient et que le paramètre de ventilation mesuré indique simultanément une augmentation de la fréquence respiratoire et une respiration plus superficielle.

17. Système d'évaluation (1) selon l'une des revendications précédentes, comportant au moins un appareil respiratoire (2).

18. Procédé de fonctionnement d'un système d'évaluation (1) selon la revendication 1.
